# EUROPÄISCHE PATENTSCHRIFT

(11) **EP 0 738 722 B1**
(45) Veröffentlichungstag und Bekanntmachung des Hinweises auf die Patenterteilung: **25.06.2003**
(21) Anmeldenummer: 96105701.5
(22) Anmeldetag: 11.04.1996
(51) Int. Cl.: C07D 307/85, C07D 405/12, A61K 31/50, A61K 31/34

(54) **5-Amino-benzofuran-2-carbonsäurederivate**
5-Amino-benzofuran-2-carboxylic acid derivatives
Dérivés d'acides 5-amino-benzofuran-2-carboxyliques

(30) Priorität: 20.04.1995 DE 19514567
(43) Veröffentlichungstag der Anmeldung: 23.10.1996
(60) Teilanmeldung: 02006144.6
(73) Patentinhaber: MERCK PATENT GmbH, 64293 Darmstadt (DE)
(72) Erfinder: Bathe, Andreas, Dr., 64283 Darmstadt (DE); Helfert, Bernd, 64372 Ober-Ramstadt (DE); Böttcher, Henning, Dr., 64287 Darmstadt (DE); Schuster, Kurt, 64372 Darmstadt (DE)

(56) Entgegenhaltungen:
- WO-A-95/33720
- DE-A- 4 333 254
- COLLECTION OF CZECHOSLOVAK CHEMICAL COMMUNICATIONS, Bd. 38, Nr. 4, 1973, PRAGUE CS, Seiten 1165-1167, XP002009856 J. KAHOVCOV ET A.: "Natural and synthetic materials with insect hormone activity. XVI. The synthesis of derivatives of oxygen heterocycles containing N-geranylaniline moiety"
- HELVETICA CHIMICA ACTA, Bd. 31, 1948, BASEL CH, Seiten 75-77, XP002009857 H. ERLENMEYER ET AL.: "Zur Kenntnis des 5-Aminocumarons"
- JOURNAL OF ORGANIC CHEMISTRY, Bd. 28, Nr. 10, Oktober 1963, EASTON US, Seiten 2744-2746, XP002009858 P.K. RAMACHANDRAN ET AL.: "The synthesis of Euparin and Dehydrotremetone"

## Beschreibung

Die Erfindung betrifft Benzofurane der Formel I 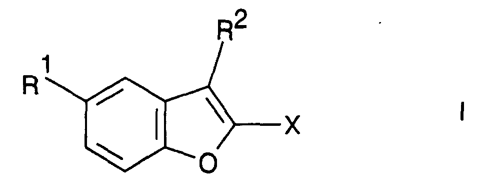 worin
- R¹: 4-R³-piperazinyl,
- R²: H, Cl, Br, OH oder OA,
- R³: Benzyl oder eine Aminoschutzgruppe, ausgewählt aus der Gruppe bestehend aus von aliphatischen, araliphatischen, aromatischen oder heterocyclischen Carbonsäuren oder Sulfonsäuren abgeleiteten Acylgruppen, Alkoycarbonyl-, Aryloxycarbonyl- und Aralkoxycarbonylgruppen, Aryl-, Aralkoxymethyl- und Aralkylgruppen,
- X: CN, COOH, COOA, COOPh, COOCH₂Ph, COOPy, CONR⁴R⁵ oder CO-Het,
- R⁴ und R⁵: jeweils unabhängig voneinander H, A oder Benzyl,
- A: Alkyl mit 1-4 C-Atomen,
- Ph: Phenyl,
- Het: Imidazol-1-yl, Triazol-1-yl oder Tetrazol-1-yl und
- Py: Pyridyl
bedeuten,
sowie deren Salze.

Ähnliche Verbindungen sind aus DE 43 33 254 bekannt.

Der Erfindung lag die Aufgabe zugrunde, neue Verbindungen zu finden, die insbesondere als Zwischenprodukte bei der Synthese von Arzneimitteln verwendet werden können, aber auch direkt zur Herstellung von Arzneimitteln Verwendung finden können.

Es wurde gefunden, daß die Verbindungen der Formel I und ihre Salze wichtige Zwischenprodukte zur Herstellung von Arzneimitteln darstellen und zugleich pharmakologische Eigenschaften besitzen. So zeigen sie beispielsweise Wirkungen auf das Zentralnervensystem.

Gegenstand der Erfindung sind die Benzofuranderivate der Formel I und ihre Salze.

Vor- und nachstehend haben die Reste R¹, R², R³, R⁴, R⁵, R⁶, X, X¹, A, Ph, Het und Py die bei den Formeln I bis VI angegebenen Bedeutungen, falls nicht ausdrücklich etwas anderes angegeben ist.

In den vorstehenden Formeln hat A 1 bis 4, vorzugsweise 1, 2, oder 3 C-Atome. A bedeutet vorzugsweise Methyl oder Ethyl, weiterhin Propyl, Isopropyl, ferner auch Butyl, Isobutyl, sek.-Butyl oder tert.-Butyl.

Der Rest Ph bedeutet Phenyl.

Der Rest Het bedeutet ein in der 1-Position substituiertes Imidazol, Triazol oder Tetrazol.

Der Rest Py bedeutet vorzugsweise einen Pyridin-2-yl-Rest, ferner auch einen Pyridin-4-yl-Rest.

Der Rest X bedeutet CN, COOH, COOA, COOPh, COOCH₂Ph, COOPy, CONR⁴R⁵ oder CO-Het.

Der Rest R¹ bedeutet einen in der 4-Position durch R³ substituierten 1-Piperazinylrest.

Der Rest R² bedeutet H, Cl, Br, OH oder OA.

Der Rest R³ bedeutet Benzyl, vorzugsweise aber eine Aminoschutzgruppe, ausgewählt aus der Gruppe bestehend aus von aliphatischen, araliphatischen, aromatischen oder heterocyclischen Carbonsäuren oder Sulfonsäuren abgeleiteten Acylgruppen, Alkoycarbonyl-, Aryloxycarbonylund Aralkoxycarbonylgruppen, Aryl-, Aralkoxymethyl- und Aralkylgruppen,.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC (tert.-Butoxycarbonyl), 2-lodethoxycarbonyl; Aralkyloxycarbonyl wie CBZ (Carbobenzoxycarbonyl, auch "Z" genannt), 4-Methoxybenzyloxycarbonyl, FMOC (9-Fluorenylmethoxycarbonyl); Arylsulfonyl wie Mtr (4-Methoxy-2,3,6-trimethylphenyl-sulfonyl). Bevorzugte Aminoschutzgruppen sind BOC und Mtr, ferner CBZ oder FMOC.

Die Verbindungen der Formel I können ein oder mehrere chirale Zentren besitzen und daher in verschiedenen stereoisomeren Formen vorkommen. Die Formel I umschließt alle diese Formen.

Gegenstand der Erfindung ist ferner ein Verfahren zur Herstellung von Benzofuranen der Formel I sowie von deren Salze, dadurch gekennzeichnet, daß man eine Verbindung, die der Formel I entspricht,
worin R¹ ein 1-Piperazinylrest ist, durch Einführung einer Aminoschutzgruppe in eine Verbindung der Formel I umwandelt, in der R¹ den 4-R³-Piperazinylrest bedeutet,
worin R³ die angegebene Bedeutung besitzt,
oder
daß man eine Verbindung, die der Formel I entspricht,
worin X eine COOA-Gruppe ist, worin A die angegenene Bedeutung besitzt,
in eine andere Verbindung der Formel I umwandelt, in der X CONR⁴R⁵ bedeutet, worin R⁴ und R⁵ die angegebenen Bedeutungen besitzen,
oder
daß man eine Verbindung, die der Formel I entspricht,
worin X eine COOH-Gruppe ist,
in eine andere Verbindung der Formel I umwandelt, in der X CO-Het bedeutet, worin Het die angegebene Bedeutung besitzt,
und/oder
daß man eine Base der Formel I durch Behandeln mit einer Säure in eines ihrer Salze umwandelt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

In den Verbindungen der Formel II bedeutet der Rest X¹ vorzugsweise Cl oder Br; er kann jedoch auch I, OH oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl-, p-Tolylsulfonyloxy, 1- oder 2-Naphthalinsulfonyloxy) bedeuten.

Die Verbindungen der Formel II sind zum Teil bekannt; die nicht bekannten Verbindungen können leicht analog zu den bekannten Verbindungen hergestellt werden.

Die Umsetzung der Verbindungen der Formel II mit Verbindungen der Formel I, in denen R¹ NH₂ bedeutet verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven.

Es ist aber auch möglich, die Verbindungen in Gegenwart eines inerten Lösungsmittels umzusetzen.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Nitroverbindungen wie Nitromethan oder Nitrobenzol; Ester wie Ethylacetat, gegebenenfalls auch Gemische der genannten Lösungsmittel untereinander oder Gemische mit Wasser.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein. Die Reaktionszeit liegt je nach den angewendeten Bedingungen zwischen einigen Minuten und 14 Tagen, die Reaktionstemperatur zwischen 0 und 150°, normalerweise zwischen 20 und 130°.

Die Umwandlung einer Verbindung der Formel I, in der R¹ eine Nitrogruppe ist, in eine Verbindung der Formel I, in der R¹ eine Aminogruppe bedeutet, erfolgt vorzugsweise mit Wasserstoffgas unter Übergangsmetallkatalyse (beispielsweise durch Hydrierung an Raney-Nickel oder Pd-Kohle in einem inerten Lösungsmittel wie Methanol oder Ethanol).

Die Umwandlung einer Verbindung der Formel I, worin R¹ ein 1-Piperazinylrest ist, in eine Verbindung der Formel I, in der R¹ den 4-R³-Piperazinylrest bedeutet, erfolgt nach bekannten Methoden, wie sie in der Literatur (z.B. in den Standardwerken wie Houben-Weyl, Methoden der organischen Chemie, Georg-Thieme-Verlag, Stuttgart); z.B für die Alkylierung oder Acylierung von Aminen beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Umwandlung einer Verbindung der Formel I, worin X eine Carboxygruppe ist, in eine Verbindung der Formel I, in der X COOA, COOPh, COOCH₂Ph, COOPy oder CO-Het bedeutet, erfolgt nach bekannten Methoden, wie sie in der Literatur (z.B. in den Standardwerken wie Houben-Weyl, Methoden der organischen Chemie, Georg-Thieme-Verlag, Stuttgart;) für derartige Veresterungen oder Amidierungen beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Umwandlung von Verbindungen der Formel I, in der X COOA bedeutet, in Verbindungen der Formel I, in der X COOH bedeutet, erfolgt z.B. mit NaOH oder KOH in Wasser, Wasser-THF oder Wasser-Dioxan bei Temperaturen zwischen 0 und 100°.

Die Umwandlung von Verbindungen der Formel I, in der X CN, COOH, COOA, COOPh, COOCH₂Ph, COOPy oder CO-Het bedeutet, in Verbindungen der Formel I, in der X CONR⁴R⁵ bedeutet, erfolgt beispielsweise mit HCONR⁴R⁵ in einem, wie oben angegebenen, inerten Lösungsmittel, gegebenenfalls unter Zusatz einer Base. Als Base dienen z.B. ein Kaliumoder Natriumalkoholat wie Kalium- oder Natriummethylat, -ethylat oder - tert.-butylat.

Die Abspaltung eines Restes R³ aus einer Verbindung der Formel I erfolgt - je nach der benutzten Schutzgruppe - z.B. mit starken Säuren, zweckmäßig mit TFA (Trifluoressigsäure) oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen liegen zweckmäßig zwischen etwa 0 und etwa 50°, vorzugsweise arbeitet man zwischen 15 und 30°.

Die Gruppe BOC wird bevorzugt mit TFA in Dichlormethan oder mit etwa 3 bis 5 n Salzsäure in Dioxan bei 15-30° abgespalten.

Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung sind auch Arzneimittel der Formel I und ihre physiologisch unbedenklichen Salze.

Gegenstand der Erfindung ist weiterhin die Verwendung der Verbindungen der Formel I als Zwischenprodukte zur Synthese von Arzneimitteln. Entsprechende Arzneimittel sind beispielsweise in DE 4333254 beschrieben.

Gegenstand der Erfindung ist dementsprechend insbesondere die Verwendung der Verbindungen der Formel I nach Anspruch 1 bei der Synthese von
1-[4-(5-Cyan-indol-3-yl)-butyl]-4-(2-carbamoyl-benzofuran-5-yl)-piperazin und dessen Salzen, dadurch gekennzeichnet,
daß man 2-(2-Formylphenoxy)-essigsäure nitriert,
daß man die so erhaltene 2-(2-Formyl-4-nitrophenoxy)-essigsäure cyclisiert,
daß man die so erhaltene 5-Nitrobenzofuran-2-carbonsäure verestert,
daß man die so erhaltene Verbindung der Formel III 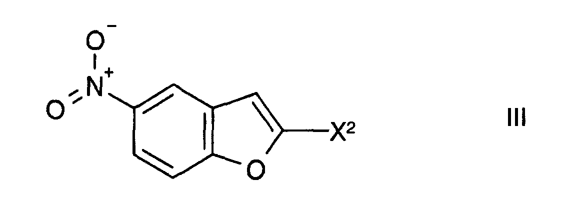 worin
- X²: COOA, COOPh oder COOCH₂Ph
bedeutet,
reduziert,
daß man die so erhaltene Verbindung der Formel I,
worin
- R¹: NH₂ und
- X²: COOA, COOPh oder COOCH₂Ph bedeutet in ihr Säureadditionssalz umwandelt und dieses mit einem Säureadditionssalz einer Verbindung der Formel II

HN(CH₂CH₂X¹)₂ II

worin
- X¹: Cl, Br, I, OH oder eine reaktionsfähig funktionell abgewandelte OH-Gruppe bedeutet,
zu einem Piperazinderivat der Formel IV 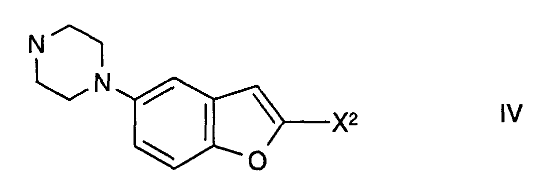 worin
- X²: COOA, COOPh oder COOCH₂Ph bedeutet umsetzt,
daß man die so erhaltene Verbindung der Formel IV
in ihr Säureadditionssalz umwandelt,
daß man dieses mit einer Verbindung umsetzt, die zur Einführung einer Aminoschutzgruppe geeignet ist,
daß man die so erhaltene Verbindung der Formel V 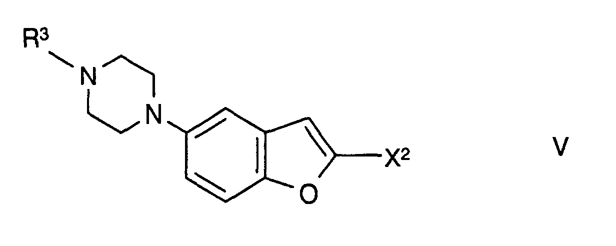 worin R³ eine Aminoschutzgruppe bedeutet und X² die angegebene Bedeutung besitzt,
in eine Verbindung der Formel VI 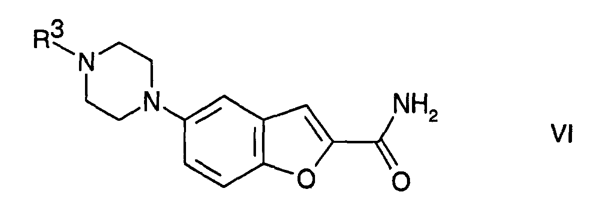 worin R³ die obige Bedeutung hat,
umwandelt,
daß man die so erhaltene Verbindung der Formel VI durch Abspaltung der Aminoschutzgruppe in 5-(1-Piperazinyl)-benzofuran-2-carboxamid oder ein Säureadditionssalz umwandelt und
daß man 5-(1-Piperazinyl)-benzofuran-2-carboxamid oder ein entsprechendes Salz mit 3-(4-Chlorbutyl)-5-cyan-indol zu 1-[4-(5-Cyan-indol-3-yl)-butyl]-4-(2-carbamoyl-benzofuran-5-yl)-piperazin umsetzt und gegebenenfalls anschließend in ein Säureadditionssalz überführt.
3-(4-Chlorbutyl)-5-cyan-indol ist bekannt aus DE 4101686, 1-[4-(5-Cyanindol-3-yl)-butyl]-4-(2-carbamoyl-benzofuran-5-yl)-piperazin ist bekannt aus DE 4333254.

Gegenstand der Erfindung ist ferner die Verwendung der Verbindungen der Formel I als Zwischenprodukte zur Synthese von Arzneimitteln, die Wirkungen auf das Zentralnervensystem zeigen.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungsund/Oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten verwendet werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel.

### Beispiel 1

1. Eine Lösung von 2,3 g 5-Nitrobenzofuran-2-carbonsäureethylester in 60 ml Methanol wird in Gegenwart von Raney-Nickel hydriert. Der Katalysator wird abfiltriert und die Lösung eingeengt. Man erhält nach üblicher Aufarbeitung 5-Aminobenzofuran-2-carbonsäureethylester, Rf 0,1 (Dichlormethan/Ethanol 9,5 : 0,5); Hydrochlorid F. 246-248°.
2. Eine Lösung von 2,05 g 5-Aminobenzofuran-2-carbonsäureethylester in 80 ml Dichlormethan wird mit 1,5 g N,N-Bis-(2-chlorethyl)-amin versetzt und 10 Stunden gerührt. Man arbeitet wie üblich auf und erhält 5-(1-Piperazinyl)-benzofuran-2-carbonsäureethylester, Rf 0,55 (Isopropanol/Wasser 95 : 5).
3. Eine Lösung von 1 g 5-(1-Piperazinyl)-benzofuran-2-carbonsäureethylester in 50 ml THF wird mit 1 g Di-tert.-butyl-dicarbonat 3 Stunden gerührt. Nach üblicher Aufarbeitung erhält man 5-(4-tert.-Butyloxycarbonyl-1-piperazinyl)-benzofuran-2-carbonsäureethylester, F. 116-118°.

### Beispiel 2

Eine Lösung von 3 g 5-(4-tert.-Butyloxycarbonyl-1-piperazinyl)-benzofuran-2-carbonsäureethylester in 100 ml N-Methylpyrrolidon wird mit 1 g Formamid und 3 g Natriumalkoholat 5 Stunden gerührt. Nach üblicher Aufarbeitung erhält man 5-(4-tert.-Butyloxycarbonyl-1-piperazinyl)-benzofuran-2-carboxamid, F. 198-200°.

### Beispiel 3

Eine Lösung von 1 g 5-(1-Piperazinyl)-benzofuran-2-carbonsäureethylester in 50 ml Dichlormethan wird mit 1 g Benzylchlorid versetzt und 2 Stunden gerührt. Nach üblicher Aufarbeitung erhält man 5-(4-Benzyl-1-piperazinyl)-benzofuran-2-carbonsäureethylester, F. 219-222°.

### Beispiel 4

1 g 5-(4-tert.-Butyloxycarbonyl-1 -piperazinyl)-benzofuran-2-carboxamid wird in 50 ml methanolischer HCI gelöst und 1 Stunde gerührt. Nach üblicher Aufarbeitung erhält man 5-(1-Piperazinyl)-benzofuran-2-carboxamid, F. 252-255°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Benzofurane der Formel I 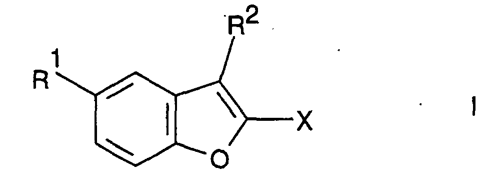 worin
R¹ 4-R³-piperazinyl,
R² H, Cl, Br, OH oder OA,
R³ Benzyl oder eine Aminoschutzgruppe, ausgewählt aus der Gruppe bestehend aus von aliphatischen, araliphatischen, aromatischen oder heterocyclischen Carbonsäuren oder Sulfonsäuren abgeleiteten Acylgruppen, Alkoycarbonyl-, Aryloxycarbonyl- und Aralkoxycarbonylgruppen, Aryl-, Aralkoxymethyl- und Aralkylgruppen,
X CN, COOH, COOA, COOPh, COOCH₂Ph, COOPy, CONR⁴R⁵ oder CO-Het,
R⁴ und R⁵ jeweils unabhängig voneinander H, A oder Benzyl,
A Alkyl mit 1-4 C-Atomen,
Ph Phenyl,
Het Imidazol-1-yl, Triazol-1-yl oder Tetrazol-1-yl und
Py Pyridyl
bedeuten,
sowie deren Salze.

2. Verbindungen nach Anspruch 1
a) 5-(4-tert.-Butyloxycarbonyl-1-piperazinyl)-benzofuran-2-carbonsäureethylester;
b) 5-(4-tert.-Butyloxycarbonyl-1-piperazinyl)-benzofuran-2-carboxamid.

3. Verfahren zur Herstellung von Benzofuranen der Formel I nach Anspruch 1 sowie von deren Salzen, **dadurch gekennzeichnet, daß** man eine Verbindung, die der Formel I entspricht,
worin R¹ ein 1-Piperazinylrest ist, durch Einführung einer Aminoschutzgruppe in eine Verbindung der Formel I umwandelt, in
der R¹ den 4-R³-Piperazinylrest bedeutet,
worin R³ die angegebene Bedeutung besitzt,
oder
daß man eine Verbindung, die der Formel I entspricht,
worin X eine COOA-Gruppe ist, worin A die angegenene Bedeutung besitzt,
in eine andere Verbindung der Formel I umwandelt, in der X CONR⁴R⁵ bedeutet, worin R⁴ und R⁵ die angegebenen Bedeutungen besitzen,
oder
daß man eine Verbindung, die der Formel I entspricht,
worin X eine COOH-Gruppe ist,
in eine andere Verbindung der Formel I umwandelt, in der X CO-Het bedeutet, worin Het die angegebene Bedeutung besitzt, und/oder
daß man eine Base der Formel I durch Behandeln mit einer Säure in eines ihrer Salze umwandelt.

4. Arzneimittel der Formel I nach Anspruch 1 und ihre physiologisch unbedenklichen Salze.

5. Verwendung der Verbindungen der Formel I nach Anspruch 1 als Zwischenprodukte zur Synthese von Arzneimitteln.

6. Verwendung der Verbindungen der Formel I nach Anspruch 1 gemäß Anspruch 5 als Zwischenprodukte zur Synthese von Arzneimitteln, die Wirkungen auf das Zentralnervensystem zeigen.

7. Verwendung der Verbindungen der Formel I nach Anspruch 1 gemäß Anspruch 5 bei der Synthese von
1-[4-(5-Cyan-indol-3-yl)-butyl]-4-(2-carbamoyl-benzofuran-5-yl)-piperazin
und dessen Salzen, **dadurch gekennzeichnet,**
**daß** man 2-(2-Formylphenoxy)-essigsäure nitriert,
**daß** man die so erhaltene 2-(2-Formyl-4-nitrophenoxy)-essigsäure cyclisiert,
**daß** man die so erhaltene 5-Nitrobenzofuran-2-carbonsäure verestert,
**daß** man die so erhaltene Verbindung der Formel III 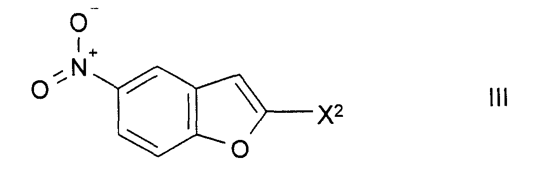 worin
X² COOA, COOPh oder COOCH₂Ph
bedeutet,
reduziert,
**daß** man die so erhaltene Verbindung der Formel I,
worin
R¹ NH₂ und
X² COOA, COOPh oder COOCH₂Ph bedeutet
in ihr Säureadditionssalz umwandelt und dieses mit einem Säureadditionssalz einer Verbindung der Formel II
HN(CH₂CH₂X¹)₂ II
worin
X¹ Cl, Br, I, OH oder eine reaktionsfähig funktionell abgewandelte OH-Gruppe bedeutet,
zu einem Piperazinderivat der Formel IV 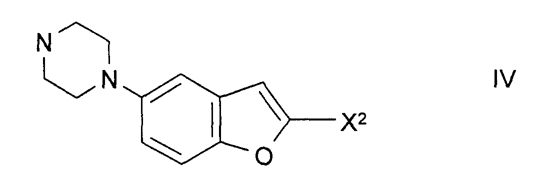 worin
X² COOA, COOPh oder COOCH₂Ph bedeutet
umsetzt,
**daß** man die so erhaltene Verbindung der Formel IV
in ihr Säureadditionssalz umwandelt,
**daß** man dieses mit einer Verbindung umsetzt, die zur Einführung einer
Aminoschutzgruppe geeignet ist,
**daß** man die so erhaltene Verbindung der Formel V 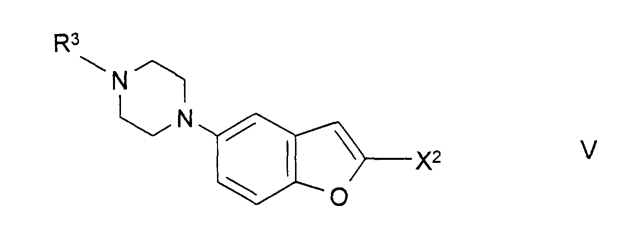 worin R³ eine Aminoschutzgruppe bedeutet und X² die angegebene Bedeutung besitzt,
in eine Verbindung der Formel VI 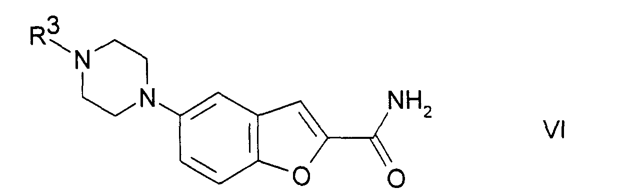 worin R³ die obige Bedeutung hat,
umwandelt,
**daß** man die so erhaltene Verbindung der Formel VI durch Abspaltung der Aminoschutzgruppe in 5-(1-Piperazinyl)-benzofuran-2-carboxamid oder ein Säureadditionssalz umwandelt, und
**daß** man 5-(1-Piperazinyl)-benzofuran-2-carboxamid mit 3-(4-Chlorbutyl)-5-cyan-indol zu 1-[4-(5-Cyan-indol-3-yl)-butyl]-4-(2-carbamoyl-benzofuran-5-yl)-piperazin umsetzt und
gegebenenfalls in sein Säureadditionssalz überführt.

8. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

9. Pharmazeutische Zubereitung, **gekennzeichnet durch** einen Gehalt an mindestens einer Verbindung der Formel I nach Anspruch 1 und/oder einem ihrer physiologisch unbedenklichen Salze.

10. Verwendung von Verbindungen der Formel I nach Anspruch 1 und ihre physiologisch unbedenklichen Salze zur Herstellung einer pharmazeutischen Zubereitung zur Bekämpfung von Krankheiten.

## Claims

1. Benzofurans of the formula I 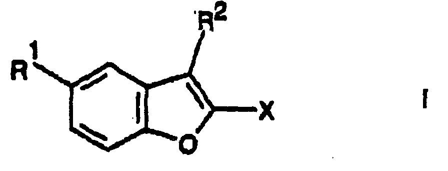 in which
R¹ is 4-R³-piperazinyl,
R² is H, Cl, Br, OH or OA,
R³ is benzyl or an amino-protecting group selected from the group consisting of acyl groups, alkoxycarbonyl, aryloxycarbonyl and aralkoxycarbonyl groups, aryl, aralkoxymethyl and aralkyl groups derived from aliphatic, araliphatic, aromatic or heterocyclic carboxylic acids or sulfonic acids,
X is CN, COOH, COOA, COOPh, COOCH₂Ph, COOPy, CONR⁴R⁵ or CO-Het,
R⁴ and R⁵ are each, independently of one another, H, A or benzyl,
A is alkyl having 1-4 carbon atoms,
Ph is phenyl,
Het is imidazol-1-yl, triazol-1-yl or tetrazol-1-yl, and
Py is pyridyl,
and salts thereof.

2. Compounds according to Claim 1
a) ethyl 5-(4-tert-butoxycarbonyl-1-piperazinyl)benzofuran-2-carboxylate;
b) 5-(4-tert-butoxycarbonyl-1-piperazinyl)benzofuran-2-carboxamide.

3. Process for the preparation of benzofurans of the formula I according to Claim 1 and salts thereof, **characterised in that**
a compound which conforms to the formula I
in which R¹ is a 1-piperazinyl radical, is converted into a compound of the formula I
in which R¹ is the 4-R³-piperazinyl radical,
in which R³ has the meaning indicated,
by introduction of an amino-protecting group,
or
**in that** a compound which conforms to the formula I
in which X is a COOA group, in which A has the meaning indicated, is converted into another compound of the formula I in which X is CONR⁴R⁵, in which R⁴ and R⁵ have the meanings indicated,
or
**in that** a compound which conforms to the formula I
in which X is a COOH group
is converted into another compound of the formula I in which X is CO-Het, in which Het has the meaning indicated,
and/or a base of the formula I is converted into one of its salts by treatment with an acid.

4. Medicament of the formula I according to Claim 1 and physiologically acceptable salts thereof.

5. Use of the compounds of the formula I according to Claim 1 as intermediates for the synthesis of medicaments.

6. Use according to Claim 5 of the compounds of the formula I according to Claim 1 as intermediates for the synthesis of medicaments which exhibit actions on the central nervous system.

7. Use according to Claim 5 of the compounds of the formula I according to Claim 1 in the synthesis of
1-[4-(5-cyanoindol-3-yl]butyl]-4-(2-carbamoylbenzofuran-5-yl)piperazine
and salts thereof, **characterised in that**
2-(2-formylphenoxy)acetic acid is nitrated,
the 2-(2-formyl-4-nitrophenoxy)acetic acid obtained in this way is cyclised,
**in that** the 5-nitrobenzofuran-2-carboxylic acid obtained in this way is esterified,
**in that** the compound of the formula III 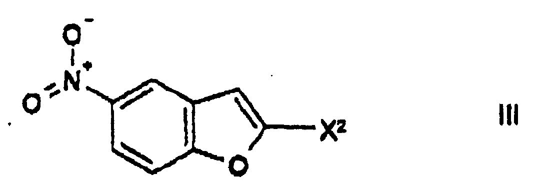 in which
X² is CODA, COOPh or COOCH₂Ph,
obtained in this way is reduced,
**in that** the compound of the formula I
in which
R¹ is NH₂, and
X² is COOA, COOPh or COOCH₂Ph,
obtained in this way is converted into its acid-addition salt, and this is reacted with an acid-addition salt of a compound of the formula II
HN(CH₂CH₂X¹)₂ II
In which X¹ is Cl, Br, I, OH or a reactively functionally modified OH group,
to give a piperazine derivative of the formula IV 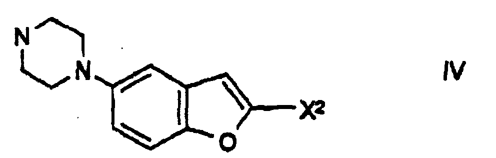 in which
X² is COOA, COOPh or COOCH₂Ph,
in that the compound of the formula IV obtained in this way is converted into its acid-addition salt,
**in that** this is reacted with a compound which is suitable for the introduction of an amino-protecting group,
**in that** the compound of the formula V 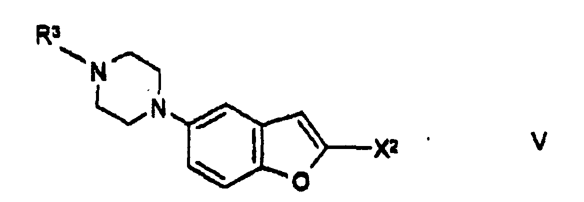 in which R³ is an amino-protecting group and X² has the meaning indicated,
obtained in this way is converted into a compound of the formula VI 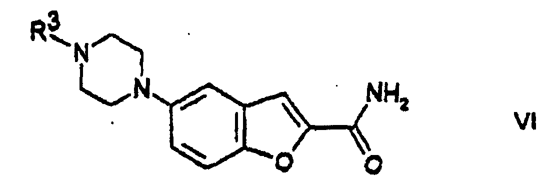 in which R³ has the above meaning,
**in that** the compound of the formula VI obtained in this way is converted into 5-(1-piperazinyl)benzofuran-2-carboxamide or an acid-addition salt by removal of the protecting group, and
**in that** 5-(1-piperazinyl)benzofuran-2-carboxamide is reacted with 3-(4-chlorobutyl)-5-cyanoindole to give 1-[4-(5-cyanoindol-3-yl)butyl]-4-(2-carbamoylbenzofuran-5-yl)piperazine,
and, if desired converted into its acid-addition salt.

8. Process for the preparation of pharmaceutical preparations, **characterised in that** a compound of the formula I according to Claim 1 and/or one of its physiologically acceptable salts is converted into a suitable dosage form together with at least one solid, liquid or semiliquid excipient or adjuvant.

9. Pharmaceutical preparation, **characterised by** a content of at least one compound of the formula I according to Claim 1 and/or one of its physiologically acceptable salts.

10. Use of compounds of the formula I according to Claim 1 and physiologically acceptable salts thereof for the preparation of a pharmaceutical preparation for combating diseases.

## Revendications

1. Benzofuranes de formule I 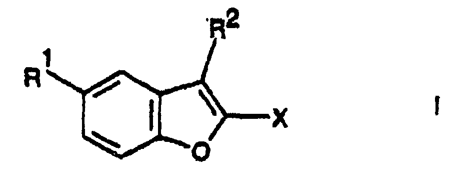 dans laquelle
R¹ représente 4-R³-pipérazinyle,
R² représente H, Cl, Br, OH ou OA,
R³ représente benzyle ou un groupement protecteur d'amine choisi parmi le groupe constitué par les groupements acyle, les groupements alkoxycarbonyle, aryloxycarbonyle et aralkoxycarbonyle, les groupements aryle, aralkoxyméthyle et aralkyle dérivés d'acides carboxyliques aliphatiques, araliphatiques, aromatiques ou hétérocycliques ou d'acides sulfoniques,
X représente CN, COOH, CODA, COOPh, COOCH₂Ph, COOPy, CONR⁴R⁵ ou CO-Hét,
R⁴ et R⁵ représentent chacun, indépendamment l'un de l'autre, H, A ou benzyle,
A représente alkyle ayant de 1 à 4 atomes de carbone,
Ph représente phényle,
Hét représente imidazol-1-yle, triazol-1-yle ou tétrazol-1-yle, et
Py représente pyridyle,
et les sels de ceux-ci.

2. Composés selon la revendication 1
a) le 5-(4-tert-butoxycarbonyl-1-pipérazinyl)benzofuran-2-carboxylate d'éthyle;
b) le 5-(4-tert-butoxycarbonyl-1-pipérazinyl)benzofuran-2-carboxamide.

3. Procédé de préparation de benzofuranes de formule I selon la revendication 1 et de sels de ceux-ci, **caractérisé en ce qu'**un composé se conformant à la formule I
dans laquelle R¹ représente un radical 1-pipérazinyle, est converti en un composé de formule I
dans laquelle R¹ représente le radical 4-R³-pipérazinyle,
dans laquelle R³ a la signification indiquée,
par l'introduction d'un groupement protecteur d'aminé,
ou
**en ce qu'**un composé se conformant à la formule I
dans laquelle X représente un groupement COOA, dans lequel A a la signification indiquée,
est converti en un autre composé de formule I dans laquelle X représente CONR⁴R⁵, dans lequel R⁴ and R⁵ ont les significations indiquées,
ou
**en ce qu'**un composé se conformant à la formule I
dans laquelle X représente un groupement COOH
est converti en un autre composé de formule I dans laquelle X représente CO-Hét, dans lequel Hét a la signification indiquée,
et/ou une base de formule I est convertie en l'un de ses sels par un traitement par un acide.

4. Médicament de formule I selon la revendication 1 et les sels physiologiquement acceptables de celui-ci.

5. Utilisation des composés de formule I selon la revendication 1 en tant qu'intermédiaires pour la synthèse de médicaments.

6. Utilisation selon la revendication 5 des composés de formule I selon la revendication 1 en tant qu'intermédiaires pour la synthèse de médicaments présentant des actions sur le système nerveux central.

7. Utilisation selon la revendication 5 des composés de formule I selon la revendication 1 dans la synthèse de
la 1-[4-(5-cyanoindol-3-yl]butyl]-4-(2-carbamoylbenzofuran-5-yl)pipérazine
et des sels de celle-ci, **caractérisée en ce que**
l'acide 2-(2-formylphénoxy)acétique est nitré,
l'acide 2-(2-formyl-4-nitrophénoxy)acétique obtenu de cette façon est cyclisé,
**en ce que** l'acide 5-nitrobenzofuran-2-carboxylique obtenu de cette façon est estérifié,
**en ce que** le composé de formule III 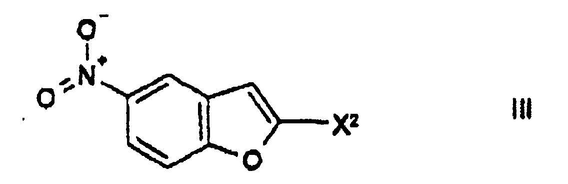 dans laquelle
X² représente CODA, COOPh ou COOCH₂Ph, obtenu de cette façon est réduit,
**en ce que** le composé de formule I
dans laquelle
R¹ représente NH₂, et
X² représente COOA, COOPh ou COOCH₂Ph,
obtenu de cette façon est converti en son sel d'addition d'acide, et ceci est réagi avec un sel d'addition d'acide d'un composé de formule II
HN(CH₂CH₂X¹)₂ II
dans laquelle X¹ représente Cl, Br, I, OH ou un groupement OH modifié de façon réactive et fonctionnelle,
pour donner un dérivé de pipérazine de formule IV 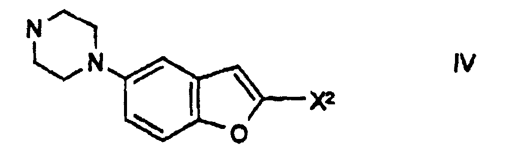 dans laquelle X² représente COOA, COOPh ou COOCH₂Ph, **en ce que** le composé de formule IV obtenu de cette façon est converti en son sel d'addition d'acide,
**en ce que** ceci est réagi avec un composé qui est convenable pour l'introduction d'un groupement protecteur d'amine,
**en ce que** le composé de formule V 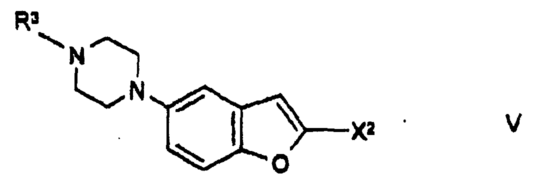 dans laquelle R³ est un groupement protecteur d'amine et X² a la signification indiquée,
obtenu de cette façon est converti en un composé de formule VI 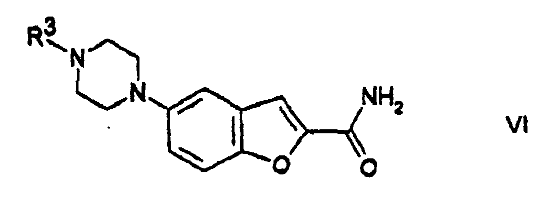 dans laquelle R³ a la signification ci-dessus,
**en ce que** le composé de formule VI obtenu de cette façon est converti en 5-(1-pipérazinyl)benzofuran-2-carboxamide ou un sel d'addition d'acide par l'élimination du groupement protecteur, et
**en ce que** le 5-(1-pipérazinyl)benzofuran-2-carboxamide est réagi avec la 3-(4-chlorobutyl)-5-cyanoindole pour donner la 1-[4-(5-cyanoindol-3-yl)butyl]-4-(2-carbamoylbenzofuran-5-yl)pipérazine, et, si on le désire, converti en son sel d'addition d'acide.

8. Procédé de préparation de préparations pharmaceutiques, **caractérisé en ce qu'**un composé de formule I selon la revendication 1 et/ou l'un de ses sels physiologiquement acceptables est mis sous une forme de dosage convenable conjointement avec au moins un excipient ou adjuvant solide, liquide ou semi-liquide.

9. Préparation pharmaceutique, **caractérisée par** une teneur en au moins un composé de formule I selon la revendication 1 et/ou l'un de ses sels physiologiquement acceptables.

10. Utilisation de composés de formule I selon la revendication 1 et de sels physiologiquement acceptables de ceux-ci pour la préparation d'une préparation pharmaceutique pour lutter contre des maladies.
